(19) Europäisches Patentamt
European Patent Office
Office européen des brevets

(11) Publication number: 0 258 055 A2

# (12) EUROPEAN PATENT APPLICATION

(21) Application number: 87307592.3

(22) Date of filing: 27.08.87

(51) Int. Cl.[4]: **A 01 N 65/00**
A 01 N 53/00, A 01 N 43/90
//(A01N65/00,53:00,37:38,
37:44),(A01N53/00,43:90),
(A01N43/90,37:44,37:38)

(30) Priority: **28.08.86 JP 200089/86**
**30.04.87 JP 104671/87**

(43) Date of publication of application:
**02.03.88 Bulletin 88/09**

(84) Designated Contracting States:
**CH DE FR GB IT LI**

(71) Applicant: **NIPPON KAYAKU KABUSHIKI KAISHA**
**11-2, Fujimi 1-chome Chiyoda-ku**
**Tokyo 102 (JP)**

(72) Inventor: **Taniguchi, Eiji**
**3-16-23 Mizutani Higashi-ku**
**Fukuoka-shi Fukuoka-ken (JP)**

**Masui, Akio**
**1-14 Minaminakano**
**Omiya-shi Saitama-ken (JP)**

**Kurozumi, Akira**
**31-14-100 Nishimine-machi**
**Ota-ku Tokyo (JP)**

**Kobayashi, Shinichi**
**2-8-2 Higashi**
**Okegawa-shi Saitama-ken (JP)**

(74) Representative: **Woods, Geoffrey Corlett et al**
**J.A. KEMP & CO. 14 South Square Gray's Inn**
**London WC1R 5EU (GB)**

(54) **A new pesticidal composition.**

(57) Disclosed herein is a pesticidal composition which comprises at least one compound selected from the group consisting of pyrethroids and synthetic pyrethroid-like compounds, and a lopseed extract or at least one compound selected from the compounds of the formula:

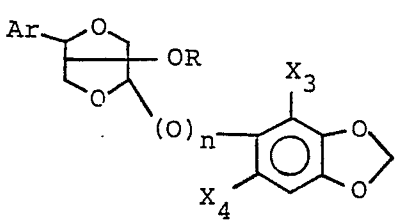

(I)

wherein $X_3$ is hydrogen or methoxy, $X_4$ is hydrogen or methoxy, R is hydrogen or acetyl, n is integer of 0 or l and Ar is

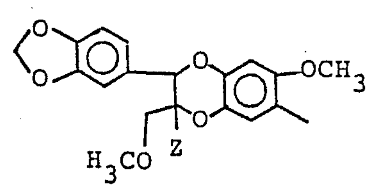

(z is hydrogen or hydroxy) or

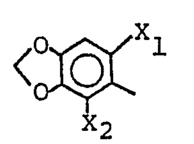

($X_1$ is hydrogen or methoxy and $X_2$ is hydrogen or methoxy).

The pesticidal composition comprising the above ingredients has a more strong and immediate pesticidal activity than the activity of the respective ingredients, and exhibits a quite excellent effect in the practical use, for example, in controlling insect pests resistant to pyrethroids.

EP 0 258 055 A2

**Description**

A NEW PESTICIDAL COMPOSITION

Detailed Description of the Invention:

The present invention relates to a new pesticidal composition which comprises

(A) at least one compound selected from the group consisting of synthetic pyrethroids and synthetic pyrethroid-like compounds and

(B) an extract obtained by

(I) extracting lopseed (Phryma leptostachya L.) with an organic solvent and removing the said solvent from the extract solution to concentrate,

(2) removing the materials which are soluble in an acidic, alkaline or neutral aqueous solution by partition of the concentrate of (I) between an adequate organic solvent and the above aqueous solution and concentrating the organic solution by removing the adequate solvent,

(3) removing the nonpolar-solvent-soluble materials

(a) by partition of the concentrate of (2) between non-polar solvent and polar solvent and concentrating the polar-solvent solution to obtain the crude extract, or

(b) by addition of the organic solution of (2) into large volume of non-polar solvent with stirring and decanting the non-polar solvent to obtain the crude extract, and if necessary

(4) isolating by chromatography technique from the crude extract of (3).

The composition of the present invention can be used as a pesticide in paddy fields, farms, orchards, etc.

Pyrethroid pesticides are now used widely for controlling agricultural and horticultural insect pests. Sesamin and piperonyl butoxide (Pb) are known for their synergistic effects in combination with these pesticides.

It was reported recently that insect pests having a reduced susceptivity to pyrethroid insecticides appeared. However, synergistic effects of ordinary synergists are yet insufficient for controlling such insect pests.

The inventors have found that a composition prepared by mixing an extract from lopseed (Phryma leptostachya L.) with at least one compound selected from the group consisting of sythetic pyrethroids and synthetic pyrethroid-like compounds has a more strong and immediate pesticidal activity than the activity of the respective components. The synergism is based on a potentiation unexpectable from the pesticidal effects of the respective ingredients. The composition exhibited a quite excellent effect in the practical use.

Synthetic pyrethroids and synthetic pyrethroid-like compounds herein include, for example, the following compounds:

α-Cyano-3-phenoxybenzyl (IRS)cis-3-(2,2-dibromovinyl)-2,2-dimethylcyclopropanecarboxylate(deltamethrin),

α-Cyano-3-phenoxybenzyl α-isopropyl-4-chlorophenylacetate(fenvalerate),

α-Cyano-3-phenoxybenzyl (IRS)-2-(2-chloro-4-trifluoromethylphenyl)amino-3-methylbutyrate(fluvalinate),

α-Cyano-3-phenoxybenzyl I-(p-ethoxyphenyl)-2,2-dichlorocyclopropanecarboxylate(cycloprothrin),

3-Phenoxybenzyl (IRS)cis, trans-3-(2,2-dichlorovinyl)-2,2-dimethylcyclopropanecarboxylate(permethrin),

α-Cyano-3-phenoxybenzyl (IRS)cis, trans-3-(2,2-dichlorovinyl)-2,2-dimethylcyclopropanecarboxylate (cypermethrin),

(RS)-α-cyano-3-phenoxybenzyl (S)-2-(4-difluoromethoxyphenyl)-3-methylbutyrate(flucythrinate),

α-Cyano-3-phenoxybenzyl 3-(2-chloro-3,3,3-trifluoropropenyl)-2,2-dimethylcyclopropanecarboxylate (cyhalothrin),

2-Methylbiphenyl-3-ylmethyl 3-(2-chloro-3,3,3-trifluoroprop-I-enyl)-2,2-dimethylcyclopropanecarboxylate(bifenthrin),

α-Cyano-4-fluoro-3-phenoxybenzyl 3-(2,2-dichlorovinyl)-2,2-dimethylcyclopropanecarboxylate(cyfluthrin),

2-(4-Ethoxyphenyl)-2-methylpropyl 3-phenoxybenzyl ether(ethofenprox).

The lopseed (Phryma leptostachya L.) extract of the present invention can be prepared, for example, as follows:

At first, whole grass, preferably root of lopseed (Phryma leptostachya L.), either raw or dried, is extracted with organic solvent of preferably I to I0 times as much as the weight of the raw or dried grass, for example, lower alcohols such as methanol and ethanol, ketones such as acetone and methyl ethyl ketone, esters such as ethyl acetate and butyl acetate, ethers such as diethyl ether and isopropyl ether, aromatic hydrocarbons such as benzene, toluene and xylene, halogenated hydrocarbons such as chloroform, methylene chloride and perchloroethylene or mixed solvents thereof, according to the conventional extracting method of plant components such as extraction with dipping or with shaking, or continuous extraction using soxhlet extractor.

After that, the organic solvent mentioned above is distilled off from the extract solution under an ordinary or reduced pressure to obtain a concentrate of adequate volume (preferably less than one-tenth of the original volume). Then, the obtained concentrate of adequate volume is subjected to partition between ① acidic, alkaline or neutral aqueous solution and ② adequate organic solution to remove the materials which are soluble in the aqueous solution of ①.

As a preferable aqueous solution, there can be mentioned 0.5 to 2% aqueous solution of mineral acids such as hydrochloric acid, or an aqueous solution of alkali, such as 2 to I0% aqueous solution of sodium hydrogen carbonate or 0.5 to 2% aqueous solution of sodium hydroxide, or water (neutral aqueous solution).

As an adequate solvent, there can be mentioned ethyl acetate, butyl acetate, diethyl ether, isopropyl ehter, benzene, toluene, xylene, p-cymene, chloroform, methylene chloride and perchloroethylene. Then the organic solvent-solution is concentrated to an adequate volume (preferably less than one-tenth of the original volume).

The concentrate obtained above is subjected to partition between a non-polar solvent and a polar solvent. Examples of the non-polar solvent include petroleum ether or aliphatic hydrocarbons ($C_{5-12}$) such as n-heptane, n-hexane, n-octane and n-undecane, and those of the polar solvent include methanol (containing preferably 0 to 20% v/v of water), ethanol (containing preferably 5 to 20% v/v of water), acetonitrile (containing preferably 0 to 20% v/v of water) and methylcellosolve (containing preferably 0 to 20% v/v of water), among which are preferably used methanol and acetonitrile. The amount of the non-polar and polar solvents used is not particularly limited but 5 to 100 times and 5 to 150 times weight, respectively, as much as the concentrate are preferable.

Further, the polar solvent-solution is separated from the phase of non-polar solvent. The solvent of the polar solvent solution is distilled off by an ordinary or reduced pressure to obtain the objective crude extract as a resinous or viscous oil.

In addition, a method for removal of the substance soluble in non-polar solvent by partition between polar and non-polar solvents may be simplified by the procedure mentioned below. Namely, while a concentrated adequate solution is added into a large quantity of a non-polar solvent under stirring, the solvent, dissolving the objective compound, comes into the solution of the non-polar solvent, and so the objective compound as such is precipitated.

The crude extracts thus obtained include the mixture of compounds of the formula:

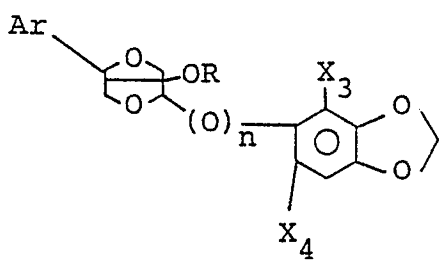

(I)

wherein $X_3$ is hydrogen or methoxy, $X_4$ is hydrogen or methoxy, R is hydrogen or acetyl, n is integer of 0 or 1 and Ar is

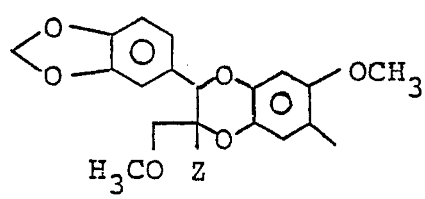

(Z is hydrogen or hydroxy) or

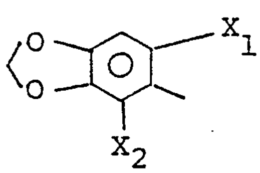

($X_1$ is hydrogen or methoxy and $X_2$ is hydrogen or methoxy).

The crude extract of the present invention further may include the analogous compounds of the formula (I) in lopseed (Phryma leptostachya L.).

The compound of the formula (I) can be separated, for example, by means of the following adsorption chromatography. A column chromatography, semi-preparative thin layer chromatography, or high-speed liquid chromatography, using silica gel, alumina or Florisil as an adsorbent are appropriate to the occasion.

As for the separation process of this invention, these chromatographies may be utilized either alone or in arbitrary combination of them. The solvent of lower polarity such as hexane, benzene or dichloromethane is preferably mixed with 5 to 95% of the solvent of higher polarity, for example, lower alcohols such as methanol and ethanol, lower ketones such as acetone and methyl ethyl ketone, acetonitrile, esters such as ethyl acetate and butyl acetate, or ethers such as diethyl ether and isopropyl ether is used as an eluent.

A column for high-speed liquid chromatography may be usually the one marketed generally, for example, Lichroprep-Si® 60 (Merck Corp.), µ-Porosil® (Waters Corp.), Resolve Si® (Waters Corp.) etc. Among the above-mentioned chromatographies for separation, an adsorption chromatography is preferable, by which a mixed solvent such as hexane/ethyl acetate or diethyl ether, or benzene/ethyl acetate or isopropyl ether is preferably used.

In addition, the crude extract can be separated also by means of partition chromatography. Namely, the fixed phase which is made of carriers such as silica gel treated by octadecylsilanol, octasilanol or phenylalkylsilanol is used. An aqueous solution containing preferably 5 to 95% of acetonitrile, methanol, ethanol, tetrahydrofuran, dioxane, etc. is used as an eluent. As a high-speed liquid chromatography with such a column for partition there may be used µ-Bondapak-$C_{18}$® (Waters Corp.), Novapak-$C_{18}$® (Waters Corp.), Radialpak-$C_8$ (Waters Corp.) or Lichrosorb RP-l8® (Merck Corp.), and as a thin-layer chromatography plate, the marketed HPTLC plate RP-l8 (Merck Corp.) may be also employed.

The pesticidal composition of the present invention preferably comprises:

(A) a mixture of the lopseed extract with at least one compound selected from the group consisting of pyrethroids and synthetic pyrethroid-like compounds, or

(B) a mixture of at least one compound of the above general formula (I) wherein Ar represents

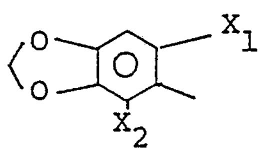

($X_1$ and $X_2$ each being a hydrogen atom or a methoxy group) with at least one compound selected from the group consisting of pyrethroids and synthetic pyrethroid-like compounds.

In the pesticidal composition of (A) of the present invention, the mixing weight ratio of the lopseed extract to the pyrethroids or synthetic pyrethroid-like compound(s) is preferably l:0.0l to l:l0, particularly l:0.l to l:5.0. In the composition of (B), the mixing weight ratio of the former to the latter is preferably l:0.0l to l:l000, particularly l:0.l to l:l0.

The pesticidal composition of the present invention is prepared as follows: according to the purpose of application, a mixture of the above-mentioned components can be used as it is or the mixture can be formulated into any convenient form such as an emulsion, wettable powder, dust, granules, aerosol, flowable powder or highly concentrated ultralow-volume spray with suitable adjuvants by a process usually employed in the art. The pesticidal composition can be used practically as it is or after dilution into a suitable concentration with water.

The pesticidal adjuvants include inert solvents and/or carriers (diluents) and various surfactants and/or organic materials. Examples of the solvents include petroleum fractions such as kerosene and gas oil; aromatic hydrocarbons such as toluene, xylene and methylnaphthalene; alcohols such as methyl cellosolve, methanol, butanol and glycol; ketones such as acetone, cyclohexanone and isophorone; amides such as dimethylformamide; sulfoxides such as dimethyl sulfoxide; mineral oils and plant oils; fatty acids; and esters such as isoamyl acetate.

Examples of the carriers (diluents) include clay, kaolin, talc, diatomaceous earth, silica, calcium carbonate, montmorillonite, bentonite, feldspar, quartz and alumina.

Examples of the surfactants include anionic, cationic, nonionic and amphoteric surfactants such as sodium salts of higher alcohol sulfates, stearyltrimethylammonium chloride, polyoxyethylene alkylphenyl ethers and laurylbetaine.

If necessary, the pesticidal composition of the present invention may contain, in addition to the carrier, usual adjuvants for pesticides such as a spreader, emulsifier, wetting agent, dispersant, fixing agent and disintegrator suitably so as to ensure the pesticidal effect.

The concentration of the mixture of the active ingredients (a mixture of ① a synthetic pyrethroid or pyrethroid-like compound with ② the above extract or a compound of the formula (I)) in the composition of the present invention is usually 0.2 to 50 wt.%, preferably 0.5 to 20 wt.%.

The composition of the present invention is used in an amount of l to 500 g/l0 a, preferably 2 to l00 g/l0 a, in terms of the active ingredients.

The pesticidal composition can be used as it is or in the form of a mixture with a germicide, herbicide, plant growth regulator, agricultural and horticultural fungicide, soil disinfectant or soil conditioner.

The insect pests on which the composition of the present invention are effective are as follows, which by no means limit the pests which can be controlled by the composition:

(l) Lepidoptera

Apple leafminer (Phyllonorycter), diamondback moth (Plutella xylostella), cotton seed worm (Promalactis inonisema), smaller tea tortix (Adoxophyes orana), soybean pod borer (Leguminivora glyoinivorella), rice leafroller (Cnapharocrocis medinalis), rice stem borer (Chilo suppressalis), oriental corn borer (Ostrinia furnacalis), cabbage armyworm (Mamesatra brassicae), rice armyworm (Pseudaletia separata), common

cutworm (Spodoptera litura), rice plant skipper (Parnara guttata), common cabbageworm (Pieris rapae crucivora), etc.

(2) Coleoptera

Cupreous chafer (Anomala cuprea), Japanese beetle (Popillia japonica), rice plant weevil (Echinocnemus squameus), rice water weevil (Lissorhoptrus oryzophilus), rice leaf beetle (Oulema oryzae), varied carpet beetle (Anthrenus verbasci), cadella (Tenebrioides mauritanicus), maize weevil (Sitophilus zeamais), 28-spotted lady beetle (Henosepilachna vigintioctopunctata), adzuki bean weevil (Callosobruchus chinensis), Japanese pine sawyer (Monochamus alternatus), cucurvit leaf beetle (Aulacophora femoralis), etc.

(3) Diptera

Tropical mosquito (Culex pipiens), yellow fever mosquito (Aedes aegypti), seed maggot (Hylemya platura), housefly (Musca domestica), melon fly (Dacus cucurbitae), rice leafminer (Agromyza oryzae), etc.

(4) Orthoptera

Mole cricket (Gryllotalpa africana), migratory locust (Locusta migratoria), short-winged rice grasshopper (Oxya japonica), German cockroach (Blattella germanica), smoky brown cockroach (Periplaneta fuliginosa) etc.

(5) Hymenoptera

Cabbage sawfly (Athalia rosae japonensis), azalea sawfly (Arge similis), etc.

(6) Acarina

Two-spotted red spider (Tetranychus urticae), citrus red mite (Panonychus citri), European red mite (Panonychus ulmi), pear rust mute (Epitrimerus pyri), tick (Haemaphysalis bispinosa), etc.

As will be shown in the following examples, the pesticidal composition of the present invention has an excellent potentiation-synergistic effect which could not be expected from the pesticidal effects of the respective components.

Now, the present invention will be described by Examples.

EXAMPLE I (Extraction)

I5 Kg of raw roots of lopseed washed with water were placed in 30 ℓ of ethyl ether in a large bottle, and immersed for 2 days at room temperature. After the ether was decanted, further 30 ℓ of ether were added and immersion was repeated. 90 ℓ of ether extract obtained by three times-immersion, were concentrated to 7 ℓ, washed three times by 2 ℓ of 5% aqueous solution of sodium hydrogen carbonate, and further washed three times by 2 ℓ of water. The obtained ether solution was dehydrated over an anhydrous sodium sulfate, and the ether was removed to obtain I20 g of a neutral fraction soluble in ether. This fraction was dissolved in 300 ml of ether, and dropped into I0 ℓ of petroleum ether at room temperature under stirring. Thus I05 g of a yellow-brown resinous precipitate (crude extract) insoluble in petroleum ether were obtained.

EXAMPLE 2 (Extraction)

I43 g (weight of raw material) of raw roots of lopseed were smashed and subjected to extraction by dipping with I ℓ of methanol for 2 days. A methanol solution obtained by three times repeated extraction was concentrated to obtain I0.5 g of a crude extract, which was applied by liquid to liquid partition with I50 ml of ether and 50 ml of water, twice. The ether layer was concentrated to 3.I4 g of crude extract, which was subjected to liquid to liquid partition with 50 ml of acetonitrile and I00 ml of petroleum ether. An acetonitrile layer thus obtained was concentrated to obtain 2.2I g of extract.

EXAMPLE 3 (Extraction)

730 g of raw roots of lopseed were dried by airing in the shade for 2 days, and extracted by dipping with 6 ℓ of ethanol for 2 days, repeatedly three times. The ethanol solution obtained was concentrated to about 200 ml, and subjected to partition by addition of 0.5 ℓ of ethyl acetate and I.0 ℓ of water. The ethyl acetate layer was washed with water and concentrated to obtain about I0 g of extract. This extract was subjected to liquid to liquid partition with 0.3 ℓ of methanol and 0.6 ℓ of n-hexane, and the obtained methanol solution was concentrated to obtain 6.6 g of extract.

EXAMPLE 4 (Separation)

I0 g of the yellow-brown resinous precipitate described in Example I, were dissolved in 40 ml of a mixed solvent of isopropylethyl/benzene = I/5 (v/v), and laid on a column (6.5 cm across, 20 cm high) packed with 400 g of Silica gel 60H (Merck Corp.) by a dry process, and separated chromatographically while varying a mixing ratio of isopropylether to benzene. Every fractions were analyzed according to high-speed liquid chromatography, as well as fractionated while assaying of pesticidal activity. (Analytical conditions of high-speed liquid chromatography:

Novapak-Silica® (Waters Corp.) 3.8 mm × I5 cm ethyl acetate/benzene = 0.5/I0 - I.0/I0 linear gradient, 0.5 ml/min UV 300 nm)

As a more insecticidaly active fraction, a part (I.0 g) eluted at Rt = I2 - I8 min according to semipreparative adsorptive high-speed liquid chromatography (Yamamura-Kagaku, YMC-Pak SH-043 (S-I5SIL), 20 mm × 25 cm) with ethyl acetate/benzene = I/I0 (0.5 ml/min) was separated. This fraction contained the compounds of the formulae (2), (3), (4), (5) and (6) described below, and so by repeated applications of high-speed liquid chromatography to the fraction, about 20 mg of the compounds (2), (3), (4), (5) and (6) were obtained respectively.

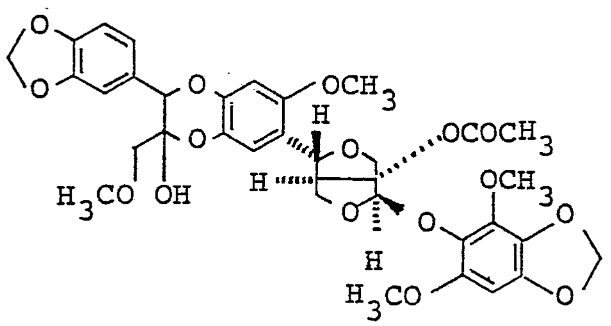

(2)

Elementary analysis:
H: 5.I6%, C: 59.92%
Calculated as molecular formula ($C_{35}H_{36}O_{16}$):
H: 5.09%, C: 58.98%
Properties:
a colourless rod crystal, melting at I38 - I40°C.
$^1$H-NMR spectrum:
2.I0 (3H, s), 2.92 (IH, m), 3.4I (3H, s), 3.45 (IH, d, J=I0), 3.5 (IH, d, J=I0), 3.74 (3H, s), 3.84 (IH, d, J=I0), 3.9 (IH, dd), 3.92 (3H, s), 4.6 (IH, dd, J=8,4), 4.67 (IH, d, J=I0), 4.74 (3H, s), 4.86 (IH, J=6), 4.95 (IH, s), 5.82 (IH, s), 5.88 (2H, s), 5.96 (2H, s), 6.24 (IH, s), 6.5I (IH, s), 6.8I (IH, d, J=8), 6.96 (IH, dd, J=8,2), 7.I2 (IH, d, J=2), 7.I8 (IH, s)

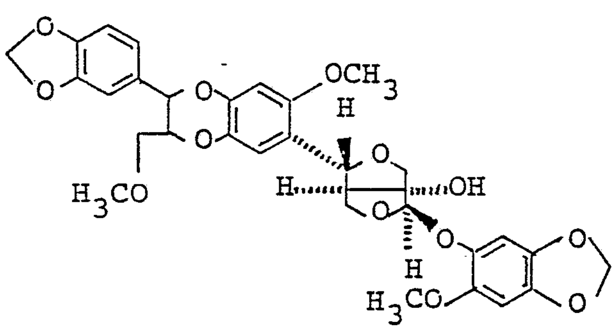

(3)

Elementary analysis:
H: 5.I3%, C:6I.53%
Calculated as molecular formula $C_{32}H_{32}O_{13}$:
H: 5.I6%, C: 6I.53%
Properties:
a colourless resinous matter
$^1$H-NMR spectrum:
I.65 (IH, s), 2.64 (IH, m), 3.3I (IH, dd, J=II,4), 3.35 (3H, s), 3.58 (IH, dd, J=II,2), 3.73 (3H, s), 3.74 (IH, d, J=9.6), 3.77 (3H, s), 4.0 (IH, m), 4.09 (IH, dd, J=9,2.5), 4.32 (IH, d, J=9.6), 4.38 (IH, dd, J=9,7.5), 4.95 (IH, d, J=6), 4.99 (IH, d, J=8), 5.I9 (IH, s), 5.90 (2H, s), 5.99 (2H, s), 6.49 (IH, s), 6.55 (IH, s), 6.79 (IH, s), 6.8I-6.96 (3H, m), 7.3I (IH, s)

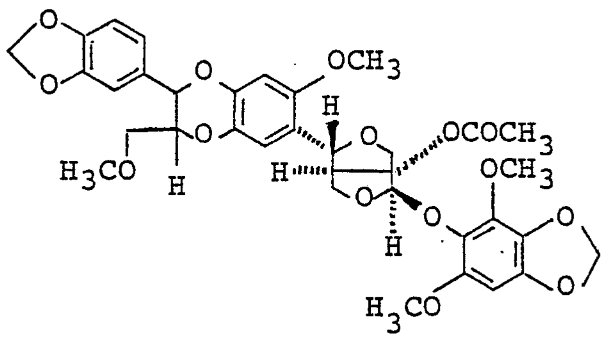

(4)

Elementary analysis:
H: 5.19%, C: 60.31%
Molecular formula:
$C_{35}H_{36}O_{15}$
Properties:
a colourless resinous matter
$^1$H-NMR spectrum:
2.10 (3H, s), 2.95 (1H, m), 3.28 (1H, dd, J=11,4), 3.35 (3H, s), 3.64 (1H, dd, J=11,2), 3.74 (6H, s), 3.76 (1H, m), 3.85 (1H, d, J=11), 3.95 (3H, s), 3.96 (1H, dd, J=10,2), 4.60 (1H, dd, J=10,6), 4.69 (1H, d, J=11), 4.90 (1H, d, J=6), 5.02 (1H, d, J=8), 5.81 (1H, s), 5.84 (2H, s), 5.96 (2H, s), 6.22 (1H, s), 6.46 (1H, s), 6.76-6.94 (3H, m), 7.17 (1H, s)

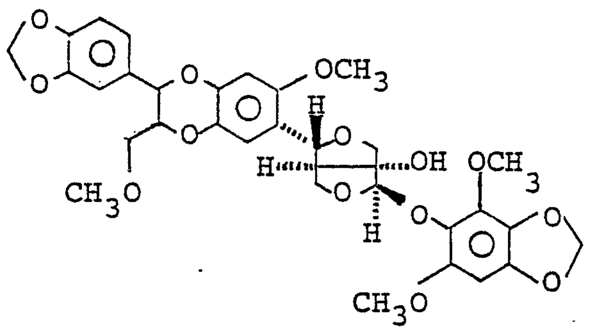

(5)

Elementary analysis:
H: 5.37%, C: 60.74%
Calculated as molecular formula $C_{33}H_{34}O_{14}$:
H: 5.23%, C: 60.55%
Properties:
a colourless crystal, melting at 158 - 159°C.
$^1$H-NMR spectrum:
1.6 (1H, s, OH), 2.64 (1H, m), 3.30 (1H, dd, J=10.7, 3.9), 3.35 (3H, s), 3.59 (1H, dd, J=10.7, 2.0), 3.73 (3H, s), 3.74 (2H, d, J=9.3), 3.79 (3H, s), 4.0 (1H, m), 4.02 (3H, s), 4.05 (2H, dd, J=8.8, 2.0), 4.30 (2H, d, J=9.3), 4.57 (2H, dd, J=8.8, 6.8), 4.94 (1H, d, J=5.9), 4.99 (1H, d, J=7.8), 5.24 (1H, s), 5.88 (2H, s), 6.00 (2H, s), 6.29 (1H, s), 6.49 (1H, s), 6.83-6.93 (3H, m), 7.31 (1H, s)

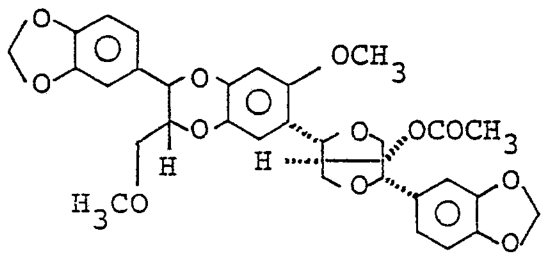

(6)

Elementary analysis:
H: 5.22%, C: 63.84%
Calculated as molecular formula $C_{33}H_{32}O_{12}$:
H: 5.20%, C: 63.86%
Properties:
a colourless resinous matter
$^1$H-NMR spectrum:
1.72 (3H, s), 3.14 (1H, m), 3.26 (1H, dd, J=11,4), 3.32 (3H, s), 3.58 (1H, dd, J=11,2), 3.76 (3H, s), 3.88 (1H, dd, J=10,4), 3.96 (1H, m), 4.28 (1H, d, J=10), 4.39 (1H, d, J=10), 4.45 (1H, dd, J=10,7), 4.92 (1H, s), 4.96 (1H, d, J=8), 4.98 (1H, d, J=5), 5.92 (2H, s), 5.96 (2H, s), 6.48 (1H, s), 6.65-6.92 (6H, m), 7.10 (1H, s)

EXAMPLE 5 (Separation)

10 g of the yellow-brown resinous precipitate described in Example 1, were dissolved in 40 ml of a mixed solvent of ethyl acetate/dichloromethane = 1/7.5 (v/v), laid on a column (4 cm across, 20 cm high) packed with 400 g of Silica gel 60 (Merck Corp.) (230 to 400 mesh) by a wet process, and separated by chromatography, while varying a mixing ratio of ethyl acetate to dichloromethane.

An insecticidal fraction was concentrated and dissolved in acetonitrile, then according to high-speed partition liquid chromatography (Waters Corp., Novapak® $C_{18}$ 39 mm × 15 cm) with $MeCN/H_2O$ = 1/1 (1.0 ml/min, UV 300 nm), a part eluted at Rt = 10 to 15 min was separated. This fraction contained the compounds (2), (3), (4), (5) and (6) and was further applied to semipreparative adsorptive high-speed liquid chromatography to obtain about 20 mg of each compounds of the formulae (2), (3), (4), (5) and (6).

In addition, data of $^1$H-NMR of Examples 2 - 6 show a chemical shift (ppm) observed in $CDCl_3$ chloroform by JEOL, FX-100.

EXAMPLE 6 (Separation)

1.6 g of the lopseed extract prepared as described above was dissolved in 3.5 ml of a mixture of benzene and ethyl acetate (10:1) and the solution was placed on a column having a diameter of 30 mm and length of 150 mm packed with Kieselgel 60 (Art. 9385) of Merck Corp. using a flash chromatograph (a product of Tokyo Rika Co.). The eluate was divided into fractions having benzene/ethyl acetate ratio of from 20:1 to 100% ethyl acetate. The components in the respective fractions were analyzed according to high-performance liquid chromatography (column: Microporasil® 3.9 mm × 300 mm; a product of Waters Corp., eluent: benzene/ethyl acetate = 9.1 (v/v), 1.0 ml/min, detector: 490 of Waters Corp. $UV_{300\ nm}$ and $UV_{275\ nm}$). Under these high-performance liquid chromatographic conditions, Compounds 7 to 18 were eluted after the following retention times (min):

compound (8) (20.4), compound (10) (9.2), compound (12) (11.2), compound (13) (4.3), compound (18) (8.4).

The compounds (7), (9), (11), (16) and (17) were prepared by hydrolyzing the acetyl group of the above respective compound by an ordinary process. $^1$H-NMR data were determined with JEOL FX-90Q or JEOL FX-100 in $CDCl_3$ at room temperature. The values were δ-values based on TMS.

Table 1 Physical properties of 3,7-dioxabicyclo-[3,3,0]octane derivatives

| Compound | R | $X_1$ | $X_2$ | $X_3$ | $X_4$ | m.p. | Optical rotation | $^1$H-NMR (δ value) |
|---|---|---|---|---|---|---|---|---|
| 7 | H | OMe | OMe | OMe | OMe | 134-5°C | $[\alpha]^{15}_D$=+85.6° (C=1.0, $CHCl_3$) | 6.30(1H,s), 6.24(1H,s), 5.85(2H,s), 5.83(2H,s), 5.63(1H,s), 5.46(1H,d), 4.29(1H,s), 4.4-3.7 (4H), 3.97(3H,s), 3.95(3H,s), 3.80(6H,s), 3.26(1H,m) |
| 8 | MeCO | OMe | OMe | OMe | OMe | 178-9°C | $[\alpha]^{26}_D$=+28.4° (C=0.8, $CHCl_3$) | 6.21(1H,s), 6.19(1H,s), 5.95(1H,s), 5.80(4H,s), 5.26(1H,d), 4.60(1H,d), 4.27(1H,dd), 4.20(1H, d), 4-3.7(1H), 3.94(6H,s), 3.73(6H,s), ca.3.6(1H, m), 1.78(3H,s) |
| 9 | H | OMe | H | OMe | OMe | | $[\alpha]^{19}_D$=+27.6° (C=0.76, $CHCl_3$) | 7.04(1H,s), 6.47(1H,s), 6.30(1H,s), 5.88(4H,s), 5.34(1H,s), 5.10(1H,d), 4.42(1H,dd), 4.32(1H, s), 4.02(1H,d), 3.96(3H,s), 3.94(1H,dd), 3.91(1H,d), 3.76(6H,s), 2.84(1H,m) |
| 10 | MeCO | OMe | H | OMe | OMe | | $[\alpha]^{19}_D$=-19.2° (C=1.3, $CHCl_3$) | 6.99(1H,s), 6.49(1H,s), 6.20(1H,s), 5.89(2H, s), 5.89(1H,s), 5.84(2H,s), 4.99(1H,d), 4.61 (1H,d), 4.40(1H,dd), 4.19(1H,d), 3.94(1H,dd), 3.94(3H,s), 3.76(3H,s), 3.72(3H,s), 3.12(1H, m), 1.69(3H,s) |
| 11 | H | OMe | OMe | H | OMe | 172-4°C | $[\alpha]^{20}_D$=+11.0° (C=0.75, $CHCl_3$) | 7.05(1H,s), 6.54(1H,s), 6.29(1H,s), 5.90(4H, s), 5.38(1H,d), 5.22(1H,s), 4.32(1H,q), 4.17 (1H,d), 4.04(1H,d), 3.99(3H,s), 3.77(1H,q), 3.76(3H,s), 3.75(3H,s), 3.21(1H,m), 2.5(1H,br) |
| 12 | MeCO | OMe | OMe | H | OMe | | $[\alpha]^{20}_D$=+200° (C=1.92, $CHCl_3$) | 6.90(1H,s), 6.51(1H,s), 6.29(1H,s), 5.89(4H, s), 5.60(1H,s), 5.35(1H,d), 4.20-4.59(3H,m), 3.99(3H,s), 3.80(3H,s), 3.76(3H,s), 3.5-3.05 (2H,m), 1.79(3H,s) |

| Compound | R | $X_1$ | $X_2$ | $X_3$ | $X_4$ | m.p. | Optical rotation | $^1$H-NMR (δ value) |
|---|---|---|---|---|---|---|---|---|
| 13 | MeCO | H | OMe | OMe | H | | | 6.97-6.41(4H,m), 5.93(4H,s), 5.37(1H,s), 4.99 (1H,d), 4.65-3.75(4H,m), 4.04(3H,s), 4.03(3H, s), 3.1-3.3(1H,m), 1.67(3H,s) |
| 14 | MeCO | OMe | H | H | OMe | | $[\alpha]^{19}_{D}$=-29.1° (C=0.45, $CHCl_3$) | 6.94(1H,s), 6.85(1H,s), 6.51(1H,s), 6.47(1H,s), 5.90(2H,s), 5.86(2H,s), 5.37(1H,s), 4.98(1H,d), 4.60(1H,d), 4.48(1H,dd), 4.21(1H,d), 3.87(1H, dd),3.79(3H,s),3.77(3H,s),3.14(1H,m),1.71(3H,s) |
| 15 | H | OMe | OMe | H | H | | | 6.92(1H,d), 6.81(1H,d), 6.78(1H,dd), 6.26(1H, s), 5.93(2H,s), 5.86(2H,s), 5.43(1H,d), 4.88 (1H,s),4.34(1H,dd), 4.02(1H,dd), 3.98(3H,s), 3.89(1H,d), 3.76(3H,s), 3.74(1H,d), 3.28(1H, m), 2.44 (1H,s) |
| 16 | H | OMe | H | H | OMe | 136-7°C | $[\alpha]^{24}_{D}$=+171.6° (C=1.55, dioxane) | 7.48(1H,m), 5.39-6.35(4H), 6.22(6H,s), 5.06 (1H,s), 4.83(1H,s), 4.12(4H,s), 3.51(1H,s), 3.46(1H,s), 3.22(1H,s), 2.84(1H,s) |
| 17 | H | OMe | H | H | H | 98-9°C | $[\alpha]^{32}_{D}$=+180.9° (C=2.20, dioxane) | 7.14(1H,s), 6.80-6.45(3H,m),6.44(1H,s), 5.91 (2H,s), 5.89(2H,s), 5.25(1H,s), 4.91(1H,d), 4.34(1H,q), 4.32(1H,d), 4.02(1H,q), 3.72(3H, s), 3.70(1H,d), 3.05(1H,br), 2.64-2.37(1H,m) |
| 18 | MeCO | OMe | OMe | H | H | 137-9°C | | 6.45-6.74(3H,m), 6.28(1H,s), 5.88(2H,s), 5.87 (2H,s), 5.69(1H,s), 5.05(1H,d), 4.59(1H, d), 4.27-3.95(2H,m), 4.01(3H,s), 3.77(3H,s), 3.43-3.77(2H,d), 2.18(3H,s) |

| Compound | R | $X_1$ | $X_2$ | $X_3$ | $X_4$ | m.p. | Optical rotation | $^1$H-NMR (δ value) |
|---|---|---|---|---|---|---|---|---|
| 19 | H | OMe | H | OMe | OMe | | $[\alpha]^{23}_{D}$=+98.5° (C=0.7, $CHCl_3$) | 7.12(1H,s), 6.48(1H,s), 6.25(1H,s), 5.89(2H,s), 5.85(2H,s), 5.18(1H,s), 4.91(1H,d), 4.52(1H,dd), 4.31(1H,d), 4.03(1H,dd), 3.99(3H,s), 3.75(3H,s), 3.72(3H,s), 3.66(1H,d), 2.57(1H,m), 1.65(1H,s) |

In compounds 7 to 15, $R_1$, $X_1$, $X_2$, $X_3$ and $X_4$ are as follows:

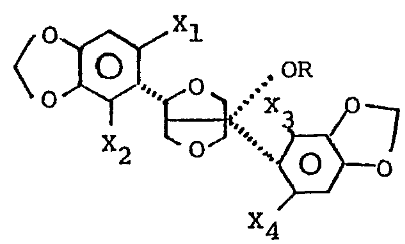

In compounds 16 to 19, $R_1$, $X_2$, $X_3$ and $X_4$ are as follows:

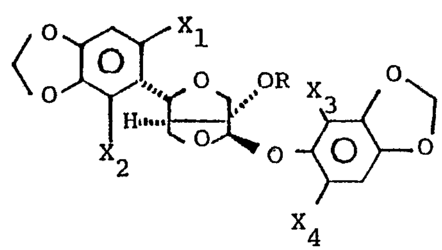

[Formulation Examples]

Formulation Example I: Emulsion

2 parts of the compound (7) was dissolved in 30% DMSO. 2 parts of permethrin, 56 parts of isophorone and I0 parts of Newkalgen I5I5-2L (a mixture of anionic and nonionic surfactant) were added to the solution to obtain an emulsion.

Formulation Example 2: Wettable powder

cycloprothrin I0 parts
compound (I0) 5 parts
white carbon I0 parts
diatomaceous earth 25 parts
clay 43 parts
Demol T®
(sodium paphthalene sulfonic acid-formaldehyde
condensate: Kao Co.) 4 parts
Newkalgen 405 H
(a mixture of anionic and nonionic surfactant:
Takemoto Fat and Oil Co., Ltd.) 3 parts

The above-mentioned components were mixed together in a mixer to obtain a homogeneous mixture, which was then hammer-milled to obtain a wettable powder.

Formulation Example 3: Dust

ethofenprox 0.5 part
compound (I4) 0.5 part
Driless A® (alkyl phospate:
Sankyo Co., Ltd.) I.0 part
white carbon 2.0 parts</DL>>clay I6.0 parts
driftless clay 80.0 parts

0.5 part of the compound (I4), 0.5 part of ethofenprox, I.0 part of Driless A, 2.0 part of white carbon and I6.0 part of clay were mixed homogeneously in a mixer. The mixture was hammer-milled. 80 parts of DL clay was added thereto and the mixture was homogeneously mixed to obtain DL dust.

Formulation Example 4: Granules

cycloprothrin 2.0 parts
compound (I7) 0.5 part
Detergent 60® (Lion Co.) I.0 part
San X p-252 (lignin sulfonic acid sodium salt
: Sanyo-Kokusaku Pulp Co., Ltd.) 2.0 parts
Kunigel V-I® (bentonite
: Kunimine Industries Co., Ltd.) 25.0 parts
clay 69.5 parts

The above-mentioned components were mixed together in a mixer to obtain a homogeneous mixture. The mixture was kneaded together with a suitable amount of water and shaped into granules with an extrusion granulating machine having a diameter of 0.8 mm. After drying, the granules were sieved to obtain I2- to 32-mesh granules.

Coated granules:

95 parts of calcium carbonate (I6- to 32-mesh) was placed in a concrete mixer. A liquid mixture of 0.5 part of Newkalgen D-4I0® (a mixture of anionic and nonionic surfactant: Takemoto Fat and Oil Co., Ltd.), 0.5 part of cycloprothrin and 2 parts of the compound (7) was added thereto under stirring. 2.0 parts of white carbon was added thereto and the mixture was thoroughly mixed to conduct coating. The granules were sieved to obtain I6- to 32-mesh granules.

Formulation Example 5: Aerosol

0.5 part of permethrin and 0.2 part of the compound (II) were dissolved in 49.3 parts of methyl cellosolve. The solution was packed in an aerosol can together with flon I2 and liquefied petroleum gas (weight ratio: 90:I0). A valve was mounted thereto to prepare an aerosol.

Example 6: Emulsion

I0 parts of the extract prepared in Example I was dissolved in 30 parts of DMSO. 2 parts of cycloprothrin, 48 parts of isophorone and I0 parts of Newkalgen I5I5-2L (a mixture of anionic and nonionic surfactant: Takemoto Fat and Oil Co., Ltd.) were added to the solution to obtain an emulsion.

Example 7: Wettable powder

| | |
|---|---|
| permethrin | I0 parts |
| extract obtained in Example I | I0 parts |
| white carbon | I0 parts |
| diatomaceous earth | 25 parts |
| clay | 38 parts |
| Demol T | 4 parts |
| Newkalgen 405H | 3 parts |

The above-mentioned components were mixed together in a mixer to obtain a homogeneous mixture, which was then hammer-milled to obtain a wettable powder.

Formulation Example 8: Dust

| | |
|---|---|
| fenvalerate | 0.5 part |
| extract obtained in Example I | 0.5 part |
| Driless A | I.0 part |
| white carbon | I.0 part |
| clay | I7.0 parts |
| DL clay | 80.0 parts |

0.5 part of fenvalerate, 0.5 part of the extract obtained in Example I, I.0 part of Driless A, I.0 part of white carbon and I7.0 parts of clay were mixed homogeneously in a mixer. The mixture was hammer-milled. 80 parts of DL clay was added thereto and the mixture was homogeneously mixed to obtain a DL dust.

Test Example I: Synergistic effect on green rice leafhopper (Nephotettix cincticeps), No.I

A pyrethroid or pyrethroid-like compound shown in the following table was diluted with acetone into a given concentration (I00 ppm of one of compounds 7 to I9 , sesamin (control) or PB was added thereto). 0.5 $\mu\ell$ of the solution was applied topically to the dorsal thorax of each of the subjects with a microsyringe. Then, the insects and young rice plants thus treated were placed in a glass cylinder, which was left to stand in a constant-temperature room at 25° C. After 24 h, the numbers of surviving insects and dead ones were counted to determine the mortality. The tests were conducted in two replications using I0 subjects in each test. The results are shown in Table 2.

Table 2 Synergistic effect on green rice leafhopper

| Pyrethroid | Dose (ppm) | Mortality (24 h after the treatment) (%) | | | | | | | | | | | | | | | |
|---|---|---|---|---|---|---|---|---|---|---|---|---|---|---|---|---|---|
| | | None | Compound | | | | | | | | | | | | | Sesamin | PB* |
| | | | (7) | (8) | (9) | (10) | (11) | (12) | (13) | (14) | (15) | (16) | (17) | (18) | (19) | | |
| None | | 0 | 0 | 0 | 0 | 0 | 0 | 0 | 0 | 0 | 0 | 0 | 0 | 0 | 0 | 0 | 0 |
| Permethrin | 100 | 100 | 100 | 100 | 100 | 100 | 100 | 100 | 100 | 100 | 100 | 100 | 100 | 100 | 100 | 100 | 100 |
| | 10 | 0 | 100 | 100 | 100 | 100 | 100 | 100 | 100 | 100 | 100 | 100 | 100 | 100 | 100 | 20 | 25 |
| | 1 | 0 | 0 | 5 | 0 | 100 | 0 | 100 | 100 | 85 | 15 | 0 | 0 | 5 | 5 | 0 | 0 |
| Fenvale-rate | 100 | 100 | 100 | 100 | 100 | 100 | 100 | 100 | 100 | 100 | 100 | 100 | 100 | 100 | 100 | 100 | 100 |
| | 10 | 75 | 100 | 100 | 100 | 100 | 100 | 100 | 100 | 100 | 100 | 100 | 100 | 100 | 100 | 100 | 95 |
| | 1 | 0 | 20 | 70 | 35 | 50 | 20 | 75 | 65 | 35 | 45 | 15 | 25 | 60 | 70 | 0 | 0 |
| Cyperme-thrin | 10 | 100 | 100 | 100 | 100 | 100 | 100 | 100 | 100 | 100 | 100 | 100 | 100 | 100 | 100 | 100 | 100 |
| | 1 | 45 | 100 | 100 | 100 | 100 | 100 | 100 | 100 | 100 | 100 | 100 | 100 | 100 | 100 | 75 | 80 |
| | 0.1 | 0 | 0 | 25 | 0 | 10 | 0 | 100 | 40 | 0 | 10 | 0 | 0 | 0 | 5 | 0 | 0 |
| Deltame-thrin | 10 | 100 | 100 | 100 | 100 | 100 | 100 | 100 | 100 | 100 | 100 | 100 | 100 | 100 | 100 | 100 | 100 |
| | 1 | 15 | 100 | 100 | 100 | 100 | 100 | 100 | 100 | 100 | 100 | 100 | 100 | 100 | 100 | 50 | 55 |
| | 0.1 | 0 | 0 | 30 | 0 | 25 | 0 | 90 | 55 | 5 | 10 | 0 | 0 | 0 | 0 | 0 | 0 |
| Cyclopro-thrin | 100 | 65 | 100 | 100 | 100 | 100 | 100 | 100 | 100 | 100 | 100 | 100 | 100 | 100 | 100 | 100 | 100 |
| | 10 | 0 | 45 | 35 | 50 | 100 | 10 | 85 | 70 | 95 | 25 | 0 | 20 | 45 | 20 | 0 | 0 |
| | 1 | 0 | 0 | 0 | 0 | 0 | 0 | 5 | 0 | 0 | 0 | 0 | 0 | 0 | 0 | 0 | 0 |

*piperonyl butoxide

| Pyrethroid | Dose (ppm) | Mortality (24 h after the treatment) (%) | | | | | | | | | | | | | | | |
|---|---|---|---|---|---|---|---|---|---|---|---|---|---|---|---|---|---|
| | | None | Compound | | | | | | | | | | | | | Sesamin | PB* |
| | | | (7) | (8) | (9) | (10) | (11) | (12) | (13) | (14) | (15) | (16) | (17) | (18) | (19) | | |
| Ethofenprox | 100 | 85 | 100 | 100 | 100 | 100 | 100 | 100 | 100 | 100 | 100 | 100 | 100 | 100 | 100 | 100 | 100 |
| | 10 | 0 | 55 | 60 | 30 | 100 | 15 | 95 | 85 | 100 | 40 | 10 | 55 | 35 | 60 | 5 | 0 |
| | 1 | 0 | 0 | 0 | 0 | 45 | 0 | 25 | 20 | 0 | 0 | 0 | 0 | 0 | 0 | 0 | 0 |
| Cyhalothrin | 10 | 100 | 100 | 100 | 100 | 100 | 100 | 100 | 100 | 100 | 100 | 100 | 100 | 100 | 100 | 100 | 100 |
| | 1 | 0 | 100 | 100 | 100 | 100 | 100 | 100 | 100 | 100 | 100 | 100 | 100 | 100 | 100 | 35 | 35 |
| | 0.1 | 0 | 5 | 0 | 10 | 25 | 0 | 10 | 15 | 0 | 5 | 0 | 10 | 0 | 0 | 0 | 0 |
| Bifenthrin | 10 | 80 | 100 | 100 | 100 | 100 | 100 | 100 | 100 | 100 | 100 | 100 | 100 | 100 | 100 | 100 | 100 |
| | 1 | 10 | 100 | 100 | 80 | 100 | 75 | 100 | 100 | 100 | 100 | 55 | 100 | 95 | 85 | 15 | 30 |
| | 0.1 | 0 | 0 | 0 | 0 | 0 | 0 | 0 | 0 | 0 | 0 | 0 | 0 | 0 | 0 | 0 | 0 |
| Cyfluthrin | 10 | 100 | 100 | 100 | 100 | 100 | 100 | 100 | 100 | 100 | 100 | 100 | 100 | 100 | 100 | 100 | 100 |
| | 1 | 0 | 80 | 100 | 100 | 100 | 100 | 100 | 100 | 100 | 100 | 100 | 100 | 100 | 100 | 45 | 20 |
| | 0.1 | 0 | 0 | 15 | 10 | 65 | 50 | 45 | 0 | 15 | 0 | 5 | 0 | 0 | 0 | 0 | 0 |
| Flucy-thrinate | 10 | 100 | 100 | 100 | 100 | 100 | 100 | 100 | 100 | 100 | 100 | 100 | 100 | 100 | 100 | 100 | 100 |
| | 1 | 0 | 100 | 100 | 100 | 100 | 100 | 100 | 100 | 100 | 100 | 100 | 100 | 100 | 100 | 30 | 55 |
| | 0.1 | 0 | 0 | 0 | 5 | 35 | 50 | 30 | 0 | 15 | 5 | 0 | 0 | 0 | 5 | 0 | 0 |

*piperonyl butoxide

Test Example 2: Synergistic effect on green rice leafhopper, No. 2

A pyrethroid compound shown in the following table was diluted with acetone into a given concentration (100 ppm of one of compounds (10), (14) and (17), sesamin (control) or PB was added thereto). 0.5 μℓ of the solution was applied topically to the dorsal thorax of each of the subjects with a microsyringe. Then, the insects and young rice plants thus treated were placed in a constant-temperature room at 25° C. After 24 h, the numbers of surviving insects and dead ones were counted to calculate the mortality. The tests were conducted in two replications using 20 subjects in each test. $LD_{50}$ (μg/g-insect) was determined from the dose and mortality according to Bliss' probit method. The coefficient of synergism was determined according to a formula of Sun & Johnson (Journal of Economic Entomology, Vol. 53, No. 5,887-892, 1960). The results are shown in Table 3.

Table 3 Synergistic effect on green rice leafhopper

| Pyrethroid | 50% Lethal dose of pyrethroid (μg/g-insect) | | | | | | | | | | |
|---|---|---|---|---|---|---|---|---|---|---|---|
| | None | Compound (10) | | Compound (14) | | Compound (17) | | Sesamin | | Piperonyl butoxide | |
| | | mix | Coeffi-cient of synergism | mix | Coeffi-cient of synergism | mix | Coeffi-cient of synergism | mix | Coeffi-cient of synergism | mix | Coeffi-cient of synergism |
| Permethrin | 3.4 | 0.074 | 4595 | 0.057 | 5965 | 0.34 | 1000 | 1.4 | 243 | 1.3 | 262 |
| Fenvalerate | 0.60 | 0.051 | 1176 | 0.12 | 500 | 0.28 | 214 | 0.33 | 182 | 0.39 | 154 |
| Cypermethrin | 0.12 | 0.035 | 343 | 0.028 | 428 | 0.022 | 545 | 0.072 | 167 | 0.047 | 255 |
| Cycloprothrin | 8.4 | 0.16 | 5250 | 0.42 | 2000 | 0.66 | 1272 | 2.9 | 290 | 3.3 | 254 |
| Ethofenprox | 5.6 | 0.10 | 5000 | 0.32 | 1750 | 0.44 | 1272 | 2.5 | 224 | 2.4 | 233 |

Test Example 3: Synergistic effect on diamondback moth

0.25 $\mu\ell$ of a solution of a pyrethroid pesticide diluted into a given concentration with acetone and containing I0 to $I0^3$ ppm of the compound (I0) or (I7) or PB was applied topically to the dorsal thorax and venter of each of the fifth-instar larvae of diamond-back moths which were susceptive to chemicals (organo-phosphorus compounds, carbamates and pyrethroids) or resistant to pyrethoroids with a microsyringe. The tests were conducted in two replications using 25 larvae in each test. The larvae thus treated were placed in a plastic cup together with cabbage leaves and then left to stand in a constant-temperature room at 25°C. 24 h after the treatment, the numbers of surviving insects and dead ones were counted to determine the mortality. The results are shown in Table 4.

Table 4 Synergistic effect on diamondback moth

1. Synergistic effect on pesticide-susceptive diamondback moth

| Pyrethroid | Dose (ppm) | Mortality of diamondback moth with the following dose (%) | | | | | | | | | |
|---|---|---|---|---|---|---|---|---|---|---|---|
| | | None | Compound (10) | | | Compound (17) | | | Piperonyl butoxide | | |
| | | | $10^1$ | $10^2$ | $10^3$ | $10^1$ | $10^2$ | $10^3$ | $10^1$ | $10^2$ | $10^3$ |
| None | | 0 | 0 | 0 | 0 | 0 | 0 | 0 | 0 | 0 | 0 |
| Cycloprothrin | $10^4$ | 100 | 100 | 100 | 100 | 100 | 100 | 100 | 100 | 100 | 100 |
| | $10^3$ | 64 | 100 | 100 | 100 | 100 | 100 | 100 | 68 | 100 | 100 |
| | $10^2$ | 0 | 52 | 100 | 100 | 18 | 100 | 100 | 0 | 0 | 0 |
| Cypermethrin | $10^2$ | 98 | 100 | 100 | 100 | 100 | 100 | 100 | 100 | 100 | 100 |
| | $10^1$ | 10 | 100 | 100 | 100 | 100 | 100 | 100 | 26 | 32 | 40 |
| | 1 | 0 | 14 | 58 | 76 | 14 | 36 | 36 | 0 | 0 | 0 |
| Permethrin | $10^3$ | 100 | 100 | 100 | 100 | 100 | 100 | 100 | 100 | 100 | 100 |
| | $10^2$ | 58 | 100 | 100 | 100 | 100 | 100 | 100 | 72 | 100 | 100 |
| | $10^1$ | 0 | 44 | 100 | 100 | 16 | 100 | 100 | 0 | 0 | 0 |

2. Synergistic effect on pesticide-resistant diamondback moth

| Pyrethroid | Dose (ppm) | Mortality of diamondback moth with the following dose (%) | | | | | | | | | |
|---|---|---|---|---|---|---|---|---|---|---|---|
| | | None | Compound (10) | | | Compound (17) | | | Piperonyl butoxide | | |
| | | | $10^1$ | $10^2$ | $10^3$ | $10^1$ | $10^2$ | $10^3$ | $10^1$ | $10^2$ | $10^3$ |
| None | | 0 | 0 | 0 | 0 | 0 | 0 | 0 | 0 | 0 | 0 |
| Cycloprothrin | $10^4$ | 42 | 100 | 100 | 100 | 100 | 100 | 100 | 36 | 46 | 100 |
| | $10^3$ | 0 | 0 | 100 | 100 | 0 | 100 | 100 | 0 | 0 | 0 |
| | $10^2$ | 0 | 0 | 60 | 100 | 0 | 16 | 100 | 0 | 0 | 0 |
| Cypermethrin | $10^2$ | 12 | 100 | 100 | 100 | 100 | 100 | 100 | 28 | 24 | 100 |
| | $10^1$ | 0 | 0 | 100 | 100 | 0 | 100 | 100 | 0 | 0 | 0 |
| | 1 | 0 | 0 | 0 | 0 | 0 | 0 | 0 | 0 | 0 | 0 |
| Permethrin | $10^3$ | 32 | 100 | 100 | 100 | 100 | 100 | 100 | 70 | 74 | 100 |
| | $10^2$ | 0 | 0 | 100 | 100 | 0 | 100 | 100 | 0 | 0 | 0 |
| | $10^1$ | 0 | 0 | 88 | 100 | 0 | 38 | 100 | 0 | 0 | 0 |

Test Example 4: Synergistic effect on housefly

Female NS strain of houseflies susceptive to chemicals and 49SR strain of houseflies resistant to pyrethroids were anesthetized with ether. l $\mu\ell$ of a pesticide solution diluted to a given concentration with acetone was applied to the dorsal thorax of each housefly with a microapplicator.

Then, the houseflies were placed in a plastic cup together with a cotton impregnated with a sucrose solution as a feed and left to stand in a constant-temperature room at 25°C.

Two days after the treatment, the numbers of surviving houseflies and dead ones were counted to determine the mortality.

The tests were conducted in two replications using l0 houseflies in each test.

The results are shown in Table 5.

Table 5 Synergistic effect on housefly

1) NS Housefly

| Pyrethroid | Dose (µg) | Mortality (2 days after the treatment) (%) | | | | | | | | | | |
|---|---|---|---|---|---|---|---|---|---|---|---|---|
| | | None | Compound | | | | | | | | Piperonyl butoxide | |
| | | | 9 | | 11 | | 12 | | 17 | | | |
| | | | 0.1[1)] | 1.0[1)] | 0.1 | 1.0 | 0.1 | 1.0 | 0.1 | 1.0 | 0.1 | 1.0 |
| None | | 0 | 0 | 0 | 0 | 0 | 0 | 0 | 0 | 0 | 0 | 0 |
| Permethrin | 1[1)] | 75 | 100 | 100 | 100 | 100 | 100 | 100 | 100 | 100 | 80 | 100 |
| Cycloprothrin | 10 | 80 | 100 | 100 | 100 | 100 | 100 | 100 | 100 | 100 | 75 | 100 |
| | 1 | 5 | 90 | 100 | 65 | 100 | 5 | 100 | 15 | 100 | 0 | 10 |

1) dose per imago (µg)

2) 49SR Housefly

| Pyrethroid | Dose (µg) | Mortality (2 days after the treatment) (%) | | | | | | | | | | |
|---|---|---|---|---|---|---|---|---|---|---|---|---|
| | | None | Compound | | | | | | | | Piperonyl butoxide | |
| | | | 9 | | 11 | | 12 | | 17 | | | |
| | | | 0.1[1)] | 1.0[1)] | 0.1 | 1.0 | 0.1 | 1.0 | 0.1 | 1.0 | 0.1 | 1.0 |
| None | | 0 | 0 | 0 | 0 | 0 | 0 | 0 | 0 | 0 | 0 | 0 |
| Permethrin | 1[1)] | 20 | 100 | 100 | 100 | 100 | 100 | 100 | 100 | 100 | 25 | 30 |
| Cycloprothrin | 10 | 5 | 100 | 100 | 100 | 100 | 100 | 100 | 100 | 100 | 5 | 5 |
| | 1 | 0 | 10 | 100 | 5 | 100 | 5 | 100 | 0 | 100 | 5 | 5 |

1) dose per imago (µg)

Test Example 5: Synergistic effect on larvae of cupreous chafer

A mixture of dry leaf mold (powder) and dry farm soil in a ratio of 3:l was used in the tests. The emulsion prepared in Example l was diluted into a given concentration with well water and 500 ml thereof [containing l00 ppm of any of compounds (l0), (l2) and (20), sesamin (control) or PB] was mixed homogeneously with l kg of the soil and the mixture was stored in a plastic box. The soil thus treated was placed in 4 plastic cups each in an amount of l50 g and 5 first-instar larvae of cupreous chafer (3 to 5 days old) were released in each cup on that day. The cup was covered and kept in a constant-temperature room at 25°C. After two days, the number of the dead larvae was counted to determine the mortality. Further, 2 weeks after the treatment, the treated soil was placed in 4 plastic cups each in an amount of l50 g and 5 first-instar larvae were released in each cup. After two days, the number of the dead larvae was counted to determine the mortality. The results are shown in Table 6.

Table 6 Synergistic effect on larvae of cupreous chafer

| Pyrethroid | Conc. (ppm) | Mortality (2 days after release) (%) | | | | | | | | | | | |
|---|---|---|---|---|---|---|---|---|---|---|---|---|---|
| | | None | | Compound (10) | | Compound (13) | | Compound (18) | | Sesamin | | *PB | |
| | | Day of treatment[1)] | 2 weeks[2)] | Day of treatment | 2 weeks | Day of treatment | 2 weeks | Day of treatment | 2 weeks | Day of treatment | 2 weeks | Day of treatment | 2 weeks |
| None | | 0 | 0 | 0 | 0 | 0 | 0 | 0 | 0 | 0 | 0 | 0 | 0 |
| Cycloprothrin | 100 | 80 | 0 | 100 | 100 | 100 | 100 | 100 | 85 | 100 | 0 | 100 | 0 |
| | 10 | 0 | 0 | 100 | 40 | 100 | 65 | 100 | 20 | 0 | 0 | 0 | 0 |
| Deltamethrin | 10 | 100 | 0 | 100 | 100 | 100 | 100 | 100 | 100 | 100 | 0 | 100 | 0 |
| | 1 | 20 | 0 | 100 | 100 | 100 | 100 | 100 | 95 | 0 | 0 | 10 | 0 |
| Fluvalinate | 10 | 65 | 0 | 100 | 100 | 100 | 95 | 100 | 70 | 100 | 0 | 100 | 0 |
| | 1 | 0 | 0 | 100 | 35 | 100 | 55 | 100 | 10 | 0 | 0 | 0 | 0 |

*PB: piperonyl butoxide

1): The larvae were released on the day of the treatment

2): The larvae were released 2 weeks after the treatment

Test Example 6: Insecticidal effect on green rice leafhopper

0.5 $\mu\ell$ of a test solution diluted to a given concentration with acetone was topically applied to the dorsal thorax of two strains of green rice leafhopper having different susceptivity to pyrethroids with a microsyringe. The insects thus treated were placed in a glass cylinder together with young rice plants and covered with a powder paper. The cylinder was kept in a constant-temperature room at 25°C. 48 h after the treatment with the pesticide, the numbers of the surviving insects and dead ones were counted to determine the mortality.

The test results are shown in Table 7.

Table 7 Pesticidal activity of mixture of pyrethroid with extract prepared in Example 1

| Pyrethroid | conc. (ppm) | Mortality of green rice leafhopper (%) | | | | $LD_{50}$ (µg/g) | | | |
|---|---|---|---|---|---|---|---|---|---|
| | | Ageo | | Izumi | | Ageo | | Izumi | |
| | | Extract-free* | Containing extract** | Extract-free* | Containing extract** | Extract-free* | Containing extract** | Extract free* | Containing extract** |
| Cycloprothin | 400 | 100 | 100 | 100 | 100 | 8.9 | 0.3 | 5.0 | 0.4 |
| | 100 | 94 | 100 | 81 | 100 | | | | |
| | 25 | 6 | 100 | 31 | 94 | | | | |
| | 6 | 0 | 60 | 0 | 25 | | | | |
| | 1.5 | 0 | 0 | 0 | 7 | | | | |
| Permethrin | 400 | 100 | 100 | 100 | 100 | 7.0 | 0.9 | 6.8 | 1.1 |
| | 100 | 64 | 100 | 57 | 100 | | | | |
| | 25 | 12 | 97 | 25 | 89 | | | | |
| | 6 | 0 | 25 | 7 | 17 | | | | |
| | 1.5 | 0 | 0 | 0 | 0 | | | | |
| None | - | 0 | 0 | 0 | 0 | - | - | - | - |

* free of the extract prepared in Example 1.

** containing 1000 ppm of the extract prepared in Example 1.

Test Example 7: Synergistic effect on green rice leafhopper

0.5 $\mu\ell$ of a test solution diluted to a given concentration with acetone was topically applied to the dorsal thorax of two strain of female green rice leafhopper having different susceptivity to pyrethroids with a microsyringe. The insects thus treated were placed in a glass cylinder together with young rice plants and covered with a powder paper. The cylinder was kept in a constant-temperature room at 25° C. One day and two days after the treatment with the pesticide, the numbers of the surviving insects and dead ones were counted to determine the mortality. The test results are shown in Table 8.

Table 8

| Strain | Days after treatment | Mortality (%) | | | | | | | | |
|---|---|---|---|---|---|---|---|---|---|---|
| | | Concentration of the extract*1) (ppm) | | | | | | | | |
| | | 100 | 80 | 60 | 40 | 20 | 10 | 8 | 6 | 0 |
| Ageo | One | 100 | 100 | 93 | 79 | 73 | 31 | 20 | 7 | 7 |
| | Two | 100 | 100 | 100 | 86 | 73 | 63 | 13 | 7 | 7 |
| Izumi | One | 100 | 100 | 100 | 100 | 87 | 60 | 53 | 27 | 25 |
| | Two | 100 | 100 | 100 | 100 | 87 | 73 | 64 | 43 | 31 |

*1) The extract of prepared by method of Example 1 with 25 ppm of cycloprothrin.

Test Example 8: Synergistic effect on larvae of diamondback moth

0.5 μℓ of a test solution diluted to a given concentration with acetone was topically applied to the dorsal thorax and venter of each of fourth-instar larvae of diamondback moth (average body weight: 3.2 mg) which was resistant to pyrethroids (collected in Panchiao, Taiwan, the Republic of China in 1983 and reared in successive generations with 10 ppm permethrin solution while selection was conducted in each generation) with a microsyringe. The larvae thus treated were placed in a plastic cup together with cabbage leaves and covered. The cup was kept in a constant-temperature room at 25°C. Two days after the treatment with the pesticide, the numbers of the surviving larvae and dead ones were counted to determine the mortality. The test results are shown in Table 9.

Table 9 Synergistic effect on larvae of diamondback moth

| Pyrethroid | (ppm) | Mortality (%) | | |
|---|---|---|---|---|
| | | Concentration of the extract*1) (ppm) | | |
| | | 1000 | 100 | 0 |
| Cycloprothrin | 100 | 100 | 50 | 14 |
| Permethrin | 100 | 100 | 67 | 7 |
| Fenvalerate | 25 | 100 | 97 | 14 |
| Fluvalinate | 25 | 100 | 84 | 7 |
| Deltamethrin | 25 | 100 | 100 | 27 |
| None | - | 0 | 0 | 0 |

*1) The extract was prepared by method of Example 1

Test Example 9: Pesticidal effects on cabbage in the fields

Cabbage seedlings from a seedbed were planted in a field on April 17. Cycloprothrin, fenvalerate, deltamethrin, the extract prepared in Example 1 or a mixture of them (formulated in the same manner as in Example 1) was diluted with 1/2000 solution of a spreader (Shingrammin) in well water. It was applied to the cabbage field in a rate of 40 liters for 10 a on May 7. The area of each experimental plot was 5.6 $m^2$. Each plot had 20 cabbages. The number of harmful insects in each plot was counted before the application and 2, 7 and 14 days after the application. The corrected density index was determined according to the following formula:

corrected density index

$$= \frac{\left(\begin{array}{l}\text{number of insects}\\ \text{after application in}\\ \text{the treated plot}\end{array}\right) \times \left(\begin{array}{l}\text{number of insects}\\ \text{before application}\\ \text{in the untreated}\\ \text{plot}\end{array}\right)}{\left(\begin{array}{l}\text{number of insects}\\ \text{before application}\\ \text{in the treated plot}\end{array}\right) \times \left(\begin{array}{l}\text{number of insects}\\ \text{after application}\\ \text{in the untreated}\\ \text{plot}\end{array}\right)} \times 100$$

The results are shown in Tables 10 and 11.

Test results:

Table 10 Effects on green caterpillar

| Pesticide | Conc. (ppm) | | Number of larvae for 20 cabbages | | | Corrected density index | | |
|---|---|---|---|---|---|---|---|---|
| | | Before application | 2 days* | 7 days* | 14 days* | 2 days* | 7 days* | 14 days* |
| Cycloprothrin | 40 | 33 | 16 | 5 | 11 | 28 | 9 | 16 |
| Fenvalerate | 40 | 26 | 8 | 1 | 3 | 18 | 2 | 5 |
| Deltamethrin | 40 | 37 | 5 | 0 | 2 | 8 | 0 | 3 |
| Extract of Example 1 | 200 | 23 | 12 | 7 | 14 | 30 | 18 | 28 |
| Cycloprothrin + extract | 20+100 | 52 | 1 | 0 | 1 | 1 | 0 | 0 |
| Deltamethrin + extract | 20+100 | 38 | 0 | 0 | 0 | 0 | 0 | 0 |
| Untreated | | 21 | 37 | 36 | 45 | 100 | 100 | 100 |

*Number of days after application

Table 11 Effects on larvae of cabbage armyworm

| Pesticide | Conc. (ppm) | | Number of larvae for 5 cabbages | | | Corrected density index | | |
|---|---|---|---|---|---|---|---|---|
| | | Before application | 2 days* | 7 days* | 14 days* | 2 days* | 7 days* | 14 days* |
| Cycloprothin | 40 | 21 | 16 | 18 | 138 | 41 | 37 | 59 |
| Fenvalerate | 40 | 28 | 4 | 5 | 38 | 8 | 4 | 12 |
| Deltamethrin | 40 | 35 | 2 | 9 | 28 | 3 | 11 | 7 |
| Extract of Example 1 | 200 | 13 | 10 | 21 | 113 | 41 | 70 | 78 |
| Cycloprothrin + extract | 20+100 | 23 | 0 | 4 | 48 | 0 | 7 | 19 |
| Fenvalerate + extract | 20+100 | 31 | 0 | 0 | 9 | 0 | 0 | 3 |
| Deltamethrin + extract | 20+100 | 16 | 0 | 0 | 6 | 0 | 0 | 3 |
| Untreated | | 31 | 58 | 72 | 344 | 100 | 100 | 100 |

* Number of days after application

**Claims**

I. A pesticidal composition which comprises

(A) at least one synthetic pyrethroid or synthetic pyrethroid-like compound and

(B) an extract obtained by

(I) extracting lopseed (Phryma leptostachya L.) with an organic solvent and removing solvent from the extract solution to provide a first concentrate,

(2) removing materials which are soluble in an acidic, alkaline or neutral aqueous solution by partition of the first concentrate between an organic solvent and said aqueous solution and separating an organic phase from an aqueous solution phase,

(3) removing nonpolar-solvent-soluble materials

(a) by concentrating the organic phase from (2) by removing organic solvent to provide a second concentrate, and partitioning the second concentrate between non-polar solvent and polar solvent, separating a non-polar solvent solution from a polar solvent solution and concentrating the polar-solvent solution to obtain a crude extract, or

(b) by addition of the organic phase from (2) into a large volume of non-polar solvent with stirring and decanting the non-polar solvent to obtain a crude extract, and optionally

(4) isolating a purified extract by chromatography technique from the crude extract of (3).

2. A pesticidal composition which comprises (A) at least one compound selected from the group consisting of

α-Cyano-3-phenoxybenzyl (IRS)cis-3-(2,2-dibromovinyl)-2,2-dimethylcyclopropanecarboxylate(deltamethrin),

α-Cyano-3-phenoxybenzyl α-isopropyl-4-chlorophenylacetate(fenvalerate),

α-Cyano-3-phenoxybenzyl (IRS)-2-(2-chloro-4-trifluoromethylphenyl)amino-3-methylbutyrate(fluvalinate),

α-Cyano-3-phenoxybenzyl I-(p-ethoxyphenyl)-2,2-dichlorocyclopropanecarboxylate(cycloprothrin),

3-Phenoxybenzyl (IRS)cis, trans-3-(2,2-dichlorovinyl)-2,2-dimethylcyclopropanecarboxylate(permethrin),

α-Cyano-3-phenoxybenzyl (IRS)cis, trans-3-(2,2-dichlorovinyl)-2,2-dimethylcyclopropanecarboxylate (cypermethrin),

(RS)- α-cyano-3-phenoxybenzyl (S)-2-(4-difluoromethoxyphenyl)-3-methylbutyrate(flucythrinate),

α-Cyano-3-phenoxybenzyl 3-(2-chloro-3,3,3-trifluoropropenyl)-2,2-dimethylcyclopropanecarboxylate(cyhalothrin),

2-Methylbiphenyl-3-ylmethyl 3-(2-chloro-3,3,3-trifluoroprop-I-enyl)-2,2-dimethylcyclopropanecarboxylate(bifenthrin),

α-Cyano-4-fluoro-3-phenoxybenzyl 3-(2,2-dichlorovinyl)-2,2-dimethylcyclopropanecarboxylate(cyfluthrin) and

2-(4-Ethoxyphenyl)-2-methylpropyl 3-phenoxybenzyl ether(ethofenprox),

and (B) at least one compound selected from the compounds of the formula:

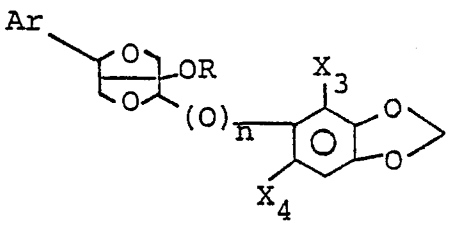

(I)

wherein $X_3$ is hydrogen or methoxy, $X_4$ is hydrogen or methoxy, R is hydrogen or acetyl, n is 0 or I and Ar is

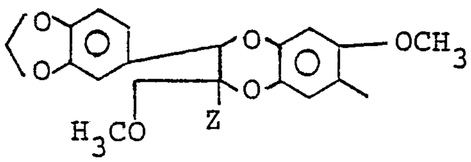

(Z is hydrogen or hydroxy) or

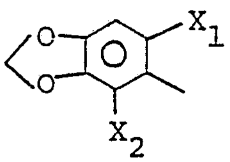

($X_1$ is hydrogen or methoxy and $X_2$ is hydrogen or methoxy) which are obtained from the extracts of Claim I.

3. A pesticidal composition which comprises (A) at least one compound selected from the group consisting of

α-Cyano-3-phenoxybenzyl (IRS)cis-3-(2,2-dibromovinyl)-2,2-dimethylcyclopropanecarboxylate(deltamethrin),

α-Cyano-3-phenoxybenzyl α-isopropyl-4-chlorophenylacetate(fenvalerate),

α-Cyano-3-phenoxybenzyl (IRS)-2-(2-chloro-4-trifluoromethylphenyl)amino-3-methylbutyrate(fluvalinate),

α-Cyano-3-phenoxybenzyl l-(p-ethoxyphenyl)-2,2-dichlorocyclopropanecarboxylate(cycloprothrin),

3-Phenoxybenzyl (IRS)cis, trans-3-(2,2-dichlorovinyl)-2,2-dimethylcyclopropanecarboxylate(permethrin),

α-Cyano-3-phenoxybenzyl (IRS)cis, trans-3-(2,2-dichlorovinyl)-2,2-dimethylcyclopropanecarboxylate (cypermethrin),

(RS)-α-cyano-3-phenoxybenzyl (S)-2-(4-difluoromethoxyphenyl)-3-methylbutyrate(flucythrinate),

α-Cyano-3-phenoxybenzyl 3-(2-chloro-3,3,3-trifluoropropenyl)-2,2-dimethylcyclopropanecarboxylate(cyhalothrin),

2-Methylbiphenyl-3-ylmethyl 3-(2-chloro-3,3,3-trifluoroprop-l-enyl)-2,2-dimethylcyclopropanecarboxylate(bifenthrin),

α-Cyano-4-fluoro-3-phenoxybenzyl 3-(2,2-dichlorovinyl)-2,2-dimethylcyclopropanecarboxylate(cyfluthrin) and

2-(4-Ethoxyphenyl)-2-methylpropyl 3-phenoxybenzyl ether(ethofenprox),

and (B) at least one compound selected from the compounds of the formula:

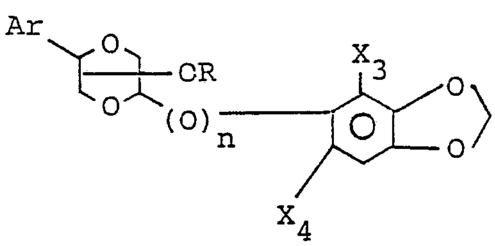

(I)

wherein $X_3$ is hydrogen or methoxy, $X_4$ is hydrogen or methoxy, R is hydrogen or acetyl, n is 0 or I and Ar is

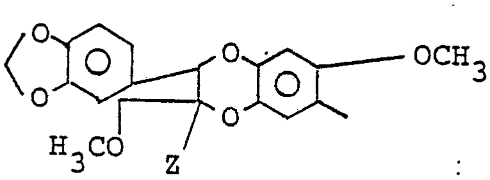

(Z is hydrogen or hydroxy) or

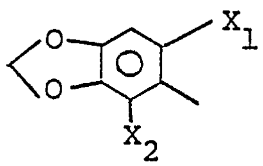

($X_1$ is hydrogen or methoxy and $X_2$ is hydrogen or methoxy).

4. A pesticidal composition according to Claim 2 or 3 wherein Ar of the compound of the formula (I) is

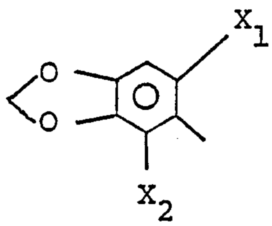

($X_1$ is hydrogen or methoxy and $X_2$ is hydrogen or methoxy).

5. A compound of the formula:

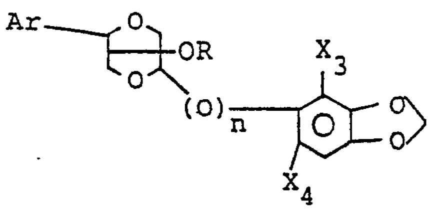

(II)

wherein Ar is

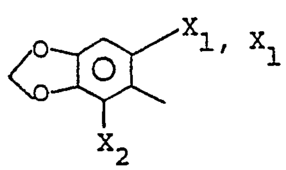

$X_1$ is hydrogen or methoxy, $X_2$ is hydrogen or methoxy, $X_3$ is hydrogen or methoxy, $X_4$ is hydrogen or methoxy, R is hydrogen or acetyl and n is 0 or l.

6. A method for obtaining ① a compound of the formula (II) as defined in claim 5 or ②, a crude extract containing the compound of the formula (II) which comprises

(l) extracting lopseed (Phryma leptostachya L.) with an organic solvent and removing the said solvent from the extract solution to provide a first concentrate,

(2) removing materials which are soluble in an acidic, alkaline or neutral aqueous solution by partition of the first concentrate between an organic solvent and said aqueous solution and separating an organic phase from an aqueous solution phase,

(3) removing nonpolar-solvent-soluble materials

(a) by concentrating the organic phase from (2) by removing organic solvent to provide a second concentrate, and partitioning the second concentrate between non-polar solvent and polar solvent, separating a non-polar solvent solution from a polar solvent solution and concentrating the polar-solvent solution to obtain a crude extract, or

(b) by addition of the organic phase from (2) into a large volume of non-polar solvent with stirring and decanting the non-polar solvent to obtain a crude extract, and optionally

(4) isolating a purified extract by chromatography technique from the crude extract of (3).

7. A method of controlling insect pests in a locus, which comprises applying to said locus a pesticidal composition as claimed in any one of claims l to 4.